# EUROPEAN PATENT APPLICATION

(11) **EP 3 360 485 A1**
(43) Date of publication of application: **15.08.2018**
(21) Application number: 18156461.8
(22) Date of filing: 13.02.2018
(51) Int. Cl.: A61B 8/06, A61B 8/08

(54) **ULTRASONIC DIAGNOSTIC APPARATUS AND CONTROL METHOD OF ULTRASONIC DIAGNOSTIC APPARATUS**

(30) Priority: 13.02.2017 KR 20170019311
(71) Applicant: Samsung Electronics Co., Ltd., Suwon-si, Gyeonggi-do (KR)
(72) Inventor: Cho, Jeong, Gangdong-gu, Seoul (KR)
(74) Representative: Gulde & Partner

(57) **Abstract**

Provided is an ultrasonic diagnostic apparatus capable of automatically correcting and changing a first position of a Doppler gate to a second position so that a user does not need to search for a position of a target. The ultrasonic diagnostic apparatus includes a probe (P); an image processor (10) configured to generate a Doppler flow imaging (120); an output portion configured to display the Doppler flow imaging generated by the image processor; and a controller (50) configured to control the probe so that the probe radiates a first ultrasonic signal based on a position of the Doppler gate (101), and the probe radiates a second ultrasonic signal to a predetermined range based on the position of the Doppler gate, and configured to move the position of the Doppler gate on the Doppler flow imaging based on the received first ultrasonic signal and second ultrasonic signal.

## Description

### BACKGROUND

### 1. Field

This disclosure relates to an ultrasonic diagnostic apparatus configured to adjust a Doppler gate position in a Doppler mode, and a control method thereof.

### 2. Description of Related Art

An ultrasonic diagnostic apparatus radiates ultrasound signals, which are generated in transducers of a probe, to an object, and acquires at least one image about a target part inside of the object (e.g., soft tissue or a blood flow) by receiving information of signals reflected from the object.

The ultrasonic diagnostic apparatus is compact, inexpensive, and capable of displaying a diagnostic imaging in real time in comparison with another type of diagnostic imaging apparatus, e.g., X-ray diagnostic apparatus, Computerized Tomography (CT) scanner, Magnetic Resonance Image (MRI) apparatus, and diagnostic nuclear medical apparatus. In addition, the ultrasonic diagnostic apparatus is safe because there is no risk of radiation exposure. Therefore, the ultrasonic diagnostic apparatus is widely used with another type of diagnostic imaging apparatus.

The ultrasonic diagnostic apparatus generates an ultrasound imaging using the reflected ultrasound signal. The ultrasound imaging may be displayed in an Amplitude mode (A mode), a Motion mode (M mode), and a Brightness mode (B mode) according to an image display method. In addition, the ultrasound imaging may be classified into a Doppler flow imaging, an extended field of view sonography (EFOVS), and a three dimensional imaging according to an image generation method.

The Doppler flow imaging is acquired by using Doppler ultrasound, which is based on the Doppler effect that the ultrasonic waves reflected from moving objects has a frequency change in proportion to their speed of motion. For example, when the transmitted ultrasound is radiated to the blood flow, particularly red blood cells, and then reflected from the blood flow, the received ultrasound varies according to the speed and direction of the red blood cells.

The Doppler mode represents a method of displaying a Doppler flow imaging. In the Doppler mode, the difference between the transmission frequency and the reception frequency, i.e., the Doppler shift frequency is accumulated as data, and the speed and direction information is converted into a color spectrum and displayed as an image.

Generally, an ultrasonic diagnostic apparatus displays a Doppler gate (or a Doppler sample point) transmitting and receiving an ultrasonic signal, at a position where a speed change is to be observed in a Doppler mode, together with Doppler flow imaging. That is, the Doppler gate is a reference for the ultrasonic diagnostic apparatus to transmit and receive ultrasonic signals and collect data.

As for an ultrasonic diagnostic apparatus in a conventional manner, there may be inconvenience such that a user manually moves a Doppler gate to a part that is not clear, on an image and it requires a fine control (e.g., mouse, touch, and trackball).

In addition, in the prior art, a user should refer a brightness mode (B mode) image to move the Doppler gate, and it is difficult to accurately control the Doppler gate when the movement of the object occurs.

### SUMMARY

It is an aspect of the disclosure to provide an ultrasonic diagnostic apparatus capable of automatically correcting and moving a position of a Doppler gate in a Doppler mode so that a user does not need to search for a position of a target by converting the Doppler mode to another mode, and so that a difficulty in correcting the position of the Doppler gate caused by a manual operation is resolved, and a control method thereof.

Additional aspects of the disclosure will be set forth in part in the description which follows and, in part, will be apparent from the description, or may be learned by practice of the invention.

In accordance with an aspect of the disclosure, an ultrasonic diagnostic apparatus include: a probe configured to radiate ultrasonic signals to an object and configured to receive reflected ultrasonic signals; an image processor configured to generate a Doppler flow imaging based on the reflected ultrasonic signals; an output portion configured to display the Doppler flow imaging and a graph of blood flow speed; and a controller configured to: control the probe so that the probe radiates a first ultrasonic signal based on a first position of a first Doppler gate to obtain a first received ultrasonic signal for a first graph of blood flow speed, control the probe so that the probe radiates a second ultrasonic signal at a predetermined range based on the first position of the first Doppler gate to obtain a second received ultrasonic signal, and based on the first received ultrasonic signal and the second received ultrasonic signal, control the probe so that the probe radiates a third ultrasonic signal based on a second position of a second Doppler gate.

The controller may be further configured to accumulate data of the first received ultrasonic signal and the second received ultrasonic signal based on a size of the first Doppler gate.

The controller may be further configured to compare the first received ultrasonic signal with the second received ultrasonic signal.

The controller may be further configured to, based on the third ultrasonic signal, obtain a third received ultrasonic signal for a second graph of blood flow speed.

The controller may be further configured to determine the second position of the second Doppler gate based a result of the comparison.

The controller may be further configured to position the second Doppler gate in a second region to which the third ultrasonic signal is radiated, and the first Doppler gate may be positioned in a first region to which the first ultrasonic signal is radiated.

The controller may be further configured to change a size of the first Doppler gate based on the second received ultrasonic signal.

The predetermined range may include a guide line including the first Doppler gate.

The output portion may be further configured to output at least one of a mark and a sound indicating that the controller has begun control the probe to radiate at the second Doppler gate.

The output portion may be further configured to output the Doppler flow imaging, and display a user interface associated with initiation of the radiation at the second Doppler gate.

In accordance with an aspect of the disclosure, a control method of an ultrasonic diagnostic apparatus may include: generating a Doppler flow imaging based on an ultrasonic signal that is radiated to an object and then reflected from the object; displaying the generated Doppler flow imaging and a representation of a first Doppler gate at a first position; and displaying a representation of a second Doppler gate on the Doppler flow imaging at a second position, wherein the second position is based on both a first ultrasonic signal associated with the first Doppler gate and a second ultrasonic signal radiated to a predetermined range with respect to the first position of the first Doppler gate.

The second ultrasonic signal may be radiated based on a size of the first Doppler gate.

The control method may further include comparing a first received ultrasonic signal associated with the first ultrasonic signal with a second received ultrasonic signal associated with the second ultrasonic signal.

The control method may further include radiating, based on the first received ultrasonic signal and the second received ultrasonic signal, the object with a third ultrasonic signal at a second Doppler gate; obtaining, based on the second Doppler gate, a third received ultrasonic signal.

The control method may further include determining a position of the second Doppler gate based on a result of the comparing.

The control method may further include positioning the second Doppler gate in a second region to which the third ultrasonic signal is radiated, wherein the first Doppler gate is positioned in a first region to which the first ultrasonic signal is radiated.

The predetermined range may include a guide line including the first Doppler gate.

The control method may further include outputting at least one of a mark and a sound when beginning to radiate at the second Doppler gate.

The displaying may include displaying a user interface associated with initiation of the radiation of the object with the third ultrasonic signal at the second Doppler gate.

### BRIEF DESCRIPTION OF THE DRAWINGS

The above and/or other aspects of the disclosure will become apparent and more readily appreciated from the following description of embodiments, taken in conjunction with the accompanying drawings of which:
FIGS. 1A, 1B, and 1C are views illustrating an ultrasonic diagnostic apparatus in accordance with an embodiment;
FIG. 2 is a block diagram illustrating a configuration of the ultrasonic diagnostic apparatus in accordance with an embodiment;
FIGS. 3 and 4 are views illustrating inconvenience of a conventional ultrasonic diagnostic apparatus;
FIGS. 5 and 6 are views illustrating a method of moving a Doppler gate by the ultrasonic diagnostic apparatus;
FIG. 7 is a view supplementing the description of FIG. 6;
FIG. 8 is a view illustrating an operation of a user interface in accordance with an embodiment;
FIG. 9 is a view illustrating an operation of a user interface in accordance with another embodiment;
FIG. 10 is a flow chart illustrating an operation in accordance with an embodiment; and.
FIGS. 11 and 12 are views illustrating an example related to the user interface.

### DETAILED DESCRIPTION

The description discloses the principles of the disclosure and discloses embodiments of the disclosure so that those skilled in the art will be able to practice the disclosure while clarifying the scope of the disclosure. The disclosed embodiments may be implemented in various forms.

Like reference numerals refer to like elements throughout the description. Well-known functions or constructions are not described in detail since they would obscure the one or more exemplar embodiments with unnecessary detail. Terms such as "part" and "portion" may be embodied as hardware or software. According to embodiments, a plurality of "part" and "portion" may be implemented as a single component or a single "part" and "portion" may include a plurality of components.

Expressions such as "at least one of," when preceding a list of elements, modify the entire list of elements and do not modify the individual elements of the list. For example, the expression, "at least one of a, b, and c," should be understood as including only a, only b, only c, both a and b, both a and c, both b and c, or all of a, b, and c.

In this description, an image may include a medical image acquired by a medical imaging device, such as a magnetic resonance imaging (MRI) apparatus, a computed tomography (CT) apparatus, an ultrasound imaging apparatus, or an x-ray imaging apparatus.

In this description, an "object" may be a person, an animal, or a part thereof, which is an object of photography. For example, an object may include a part of the body (organ) or a phantom.

In the entire description, "ultrasound imaging" refers to an image of a target object O which is generated based on ultrasonic signals transmitted to and reflected from the target object.

Hereinafter, embodiments will be described in detail with reference to the drawings.

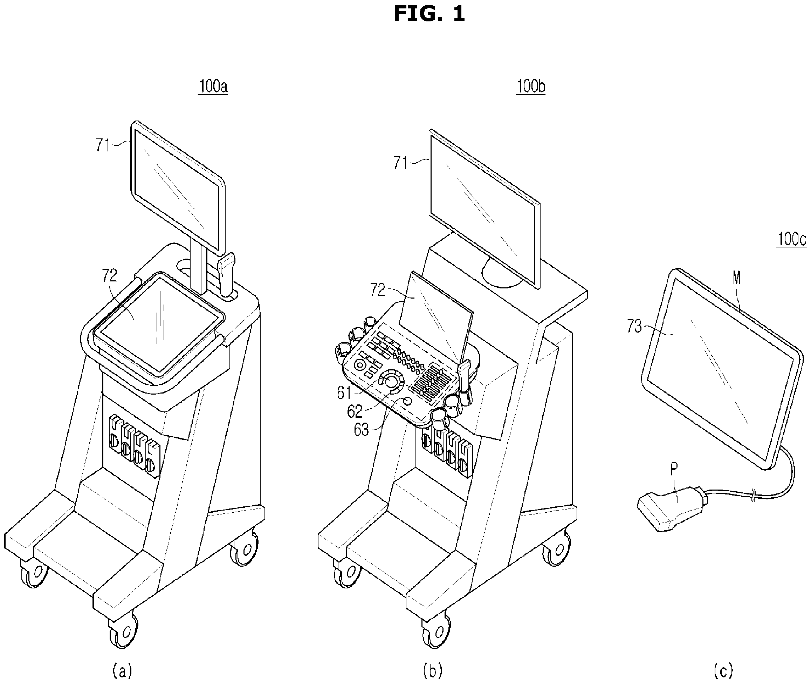

FIGS. 1A to 1C are views illustrating an ultrasonic diagnostic apparatus in accordance with an embodiment. Particularly, the disclosed ultrasonic diagnostic apparatus 100 may be implemented as various apparatuses 100a, 100b, and 100c.

Referring to FIGS. 1A and 1B, the ultrasonic diagnostic apparatus 100a and 100b may include a main display 71 and an auxiliary display 72. One of the main display 71 and the auxiliary display 72 may be implemented by a touch screen. The main display 71 and the auxiliary display 72 may display an ultrasound imaging or a variety of information processed by the ultrasonic diagnostic apparatus 100a and 100b. In addition, since the main display 71 and the auxiliary display 72 are implemented by a touch screen and provide a graphical user interface (GUI), the main display 71 and the auxiliary display 72 may receive data for controlling the ultrasonic diagnostic apparatus 100a and 100b. For example, the main display 71 may display an ultrasound imaging and the auxiliary display 72 may display a control panel for controlling the display of the ultrasound imaging in a GUI form. The auxiliary display 72 may receive data for controlling the display of the image through the control panel displayed in the GUI form. The ultrasonic diagnostic apparatuses 100a and 100b may control the display of the ultrasound imaging displayed on the main display 71 using the received control data.

Referring to FIG. 1B, the ultrasonic diagnostic apparatus 100b may further include a control panel, that is, an input 60, in addition to the main display 71 and the auxiliary display 72. The input 60 may include a button, a trackball, a jog switch, and a knob and may receive data for controlling the ultrasonic diagnostic apparatus 100b from a user. For example, the input 60 may include a trackball 61, a Time Gain Compensation (TGC) button 62, and a freeze button 63. The TGC button 62 is configured to set a TGC value for each depth of the ultrasound imaging. When the input through the freeze button 63 is detected during the scan of the ultrasound imaging, the ultrasonic diagnostic apparatus 100b may maintain a state, in which a frame image at a corresponding time is displayed. The description of the trackball 61 will be described later with reference to the drawings.

Meanwhile, a button, a track ball, a jog switch, and knob, included in the input 60 may be provided as the GUI on the main display 71 or the auxiliary display 72.

Referring to FIG. 1C, the ultrasonic diagnostic apparatus 100c may be implemented as a portable type. Examples of portable ultrasonic diagnostic apparatus 100c may include a smart phone, a laptop computer, a PDA, and a tablet PC that include a probe and an application, but is not limited thereto.

The ultrasonic diagnostic apparatus 100c may include a probe P and a main body M, wherein the probe P may be connected to one side of the main body M by wire or wirelessly. The main body M may include a display 73 including a touch screen panel. The display 73 may display an ultrasound imaging, a variety of information processed by the ultrasonic diagnostic apparatus, and a GUI.

Meanwhile, the disclosed embodiments may be embodied in the form of a computer-readable recording medium for storing commands and data executable by a computer. The command may be stored in the form of program code, and when executed by the processor, the command may generate a predetermined program module to perform a predetermined operation. In addition, when executed by a processor, the commands may perform certain operations of the disclosed embodiments.

According to an embodiment, the ultrasonic diagnostic apparatus 100 may generate a Doppler flow imaging based on the received ultrasonic signals and display a Doppler gate or a representation of a Doppler gate, which is to be observed by a user, together with the generated Doppler imaging, on the displays 71, 72 and 73. The user may input a command for automatically moving the Doppler gate through the user interface displayed on the displays 71, 72, and 73 by touching. This motion of the Doppler gate may also be referred to as finding a position of a second Doppler gate. A detailed description related thereto will be described later with reference to the drawings.

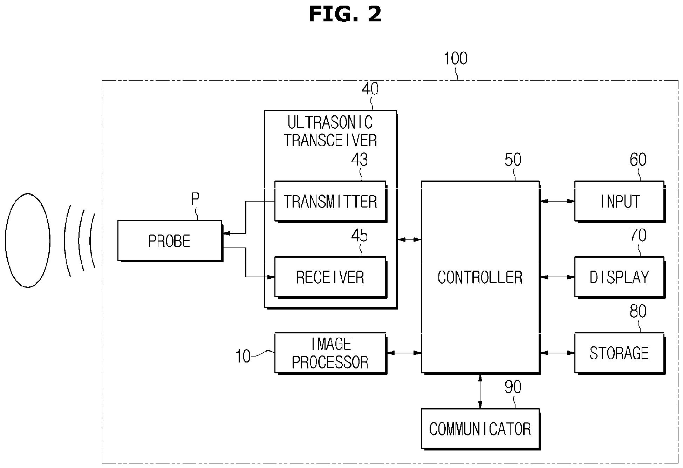

FIG. 2 is a block diagram illustrating a configuration of the ultrasonic diagnostic apparatus in accordance with an embodiment.

Referring to FIG. 2, according to an embodiment, an ultrasonic diagnostic apparatus 100 may include a probe P, an ultrasonic transceiver 40, a controller 50, an image processor 10, an output portion 70, a storage 80, a communicator 90, and an input 60.

As illustrated in FIG. 1, the ultrasonic diagnostic apparatus 100 may be implemented as a portable type as well as a cart type. Examples of portable ultrasonic diagnostic apparatus 100c may include a smart phone, a laptop computer, a PDA, and a tablet PC that include a probe and an application, but is not limited thereto.

The probe P may include a plurality of transducers. The plurality of transducers may transmit ultrasonic signals to the object O in accordance with transmission signals applied from a transmitter 43. The plurality of transducers may receive ultrasonic signals reflected from the object O and generate a reception signal. These reflections may be referred to as echoes. The probe P may be implemented integrally with the ultrasonic diagnostic apparatus 100 or may be implemented as a separate type connected to the ultrasonic diagnostic apparatus 100 by wired or wireless connection. Further, the ultrasonic diagnostic apparatus 100 may include one or a plurality of probes P according to an embodiment.

The controller 50 controls the transmitter 43 so that the transmitter 43 generates a transmission signal to be applied to each of the plurality of transducers, by considering a focus point and a position of the plurality of transducers contained in the probe P. In some embodiments, the focus point is associated with a Doppler gate.

The controller 50 controls a receiver 45 so that the receiver 45 generates ultrasound data by performing analog-to-digital conversion on signals received from the probe P, and by summing reception signals. The reception signals are converted into digital signals and summed. The summing is performed based on considering the focus point and position of the plurality of transducers.

The image processor 10 generates various ultrasound imaging using the ultrasound data generated by the receiver 45.

For example, the image processor 10 may display an image in an Amplitude mode (A mode), a Motion mode (M mode), and a Brightness mode (B mode). Particularly, the brightness mode (B mode) is a method of generating an image by indicating reflected ultrasonic signals as the brightness of the dot or given point. The brightness of a point is determined in proportion to the amplitude of the reflected signal and provides a brightness level of 256 or more. In addition, the brightness mode (B mode) may be applied as a color mode generated by assigning colors to a point in the image based on the brightness of the given point.

In addition, the image processor 10 generates a Doppler flow imaging based on the Doppler effect.

Doppler flow imaging may convert a frequency change of an ultrasonic signal at a specific position of an object, into an alternating current (AC) frequency and display it in a graph, and express a change in a flow rate of a designated region in an image of the brightness mode, in colors. By the Doppler effect, reflected signals are shifted in frequency in proportion to the velocity of the reflecting object, for example, blood cells in a blood flow within a blood vessel.

According to an embodiment, the Doppler flow imaging includes a Doppler gate configured to indicate a certain position reflecting ultrasonic signals, to a user. The Doppler gate, in some embodiments, represents a depth. Reflected waves from the ultrasonic signal emitted by the probe return to the probe as echoes after reflecting off, e.g., blood cells carried in a blood vessel. These echoes are echo data useful for determining graphs of, for example, blood flow. The user may designate a region to observe the flow rate, in the brightness mode using the Doppler gate. The depth at which the echo originates corresponds to a position of the gate. In some embodiments, the receiver 45 processes a continuum of depths, and the gate represents a corresponding range of depths, for example, a range or region spanning the width of, for example, a blood vessel. A detailed description related thereto will be described later with reference to the drawings.

The output portion 70 may perform an interaction with a user by the display 71 to 73 configured to display the generated ultrasound imaging and a variety of information processed by the ultrasonic diagnostic apparatus 100, and by a speaker configured to provide a warning sound and a variety of sounds related to an operation.

According to the implementation method, the ultrasonic diagnostic apparatus 100 may include a single display or a plurality of displays 71, 72, and 73. In addition, the displays 71, 72, and 73 may be implemented as a touch screen in combination with the touch panel.

The controller 50 may control the overall operation of the ultrasonic diagnostic apparatus 100 and a signal flow between the internal components of the ultrasonic diagnostic apparatus 100. The controller, in some embodiments, controls the overall operation by performing various operations. The controller 50 may include a memory for storing programs or data for performing the functions of the ultrasonic diagnostic apparatus 100, and a processor for processing programs or data. In addition, the controller 50 may receive a control signal from the input 60 or an external device, and then control the operation of the ultrasonic diagnostic apparatus 100.

Particularly, the controller 50 controls the image processor 10 so that the image processor 10 generates an ultrasound imaging based on ultrasonic signals transmitted from the probe P. In addition, when the generated ultrasound imaging is a Doppler flow imaging, the controller 50 may control the output portion 70 so that the output portion 70 displays a Doppler gate together with the generated Doppler flow imaging image.

The Doppler gate represents a position for observing the speed change of the moving object in the ultrasound imaging. That is, the user adjusts the Doppler gate to the position of the object corresponding to the target object, e.g., the blood flow, and the controller 50 controls the probe P to transmit and receive the ultrasonic signal based on the position and size of the Doppler gate. The position may correspond to a depth and the size may correspond to a range of depths or region. Accordingly, the controller 50 performs Doppler processing based on the received ultrasound signals and the accumulated data, and controls the image processor 10 so that the image processor 10 indicates the object in a spectrum.

Meanwhile, when an object corresponding to a target of ultrasound diagnosis, e.g., a patient, is moved, the image processor 10 may generate an ultrasound imaging that is different from a previously generated ultrasound imaging. Accordingly, the user has to move the Doppler gate (for example, reconfigure the focus of the probe and the depth or range at which echoes are observed) again, which is inconvenient.

The disclosed ultrasonic diagnostic apparatus 100, particularly, the controller 50 may automatically correct the position of the Doppler gate in the changed ultrasound imaging, thereby reducing the inconvenience that the user must manually move the Doppler gate through the input 60. In some embodiments, correction of the Doppler gate is carried out by finding a position of a second Doppler gate.

Particularly, the controller 50 radiates a plurality of ultrasonic signals (multi-beam) to a region of the object in which the Doppler gate is placed, and a certain range that is set to include the region of the object, and accumulates data related to the received ultrasound signals. That is, even if the ultrasound imaging is generated again by the movement of the patient, the controller 50 may compare the data related to the region in which the Doppler gate is previously placed, with changed data, based on the accumulated data, and move the Doppler gate to a similar pattern or identical data region. A description thereof will be described in detail with reference to the drawings.

The ultrasonic diagnostic apparatus 100 may include a communicator 90 and be connected to an external device (e.g., a server, a medical apparatus, a portable device (a smartphone, a tablet PC, and a wearable device) via the communicator 90.

The communicator 90 may include at least one component configured to communicate with an external device. For example, the communicator may include at least one of a short range communication module, a wired communication module, and a wireless communication module.

The communicator 90 may receive a control signal and data from the external device and transmit the received control signal to the controller 50 so that the controller 50 controls the ultrasonic diagnostic apparatus 100 according to the received control signal.

Alternatively, the controller 50 may transmit a control signal to the external device through the communicator 90, thereby controlling the external device according to the control signal of the controller.

For example, the external device may process data of an external device according to a control signal of a controller received through a communicator.

In the external device, a program for controlling the ultrasonic diagnostic apparatus 100 may be installed, and thus the program may include a command for performing a part or all of an operation of the controller 50.

The program may be installed in advance in an external device, or a user of the external device may download the program from a server providing the application and install the program. The server providing the application may include a recording medium in which the program is stored.

The storage 80 may store various data or programs for driving and controlling the ultrasonic diagnostic apparatus 100, input/output ultrasound data, and acquired ultrasound imaging.

The input 60 may receive a user's input for controlling the ultrasonic diagnostic apparatus 100. For example, the input of the user may include inputting for operating a button, a keypad, a mouse, a trackball, a jog switch, or a knob, touching a touch pad or a touch screen, a voice input, a motion input, and biometric information input (e.g. iris recognition and fingerprint recognition), but is not limited thereto.

In the block diagram of the ultrasonic diagnostic apparatus 100 shown in FIG. 2, at least one component may be added or deleted. It will be readily understood by those skilled in the art that the mutual position of the components can be changed by corresponding to the performance or structure of the system.

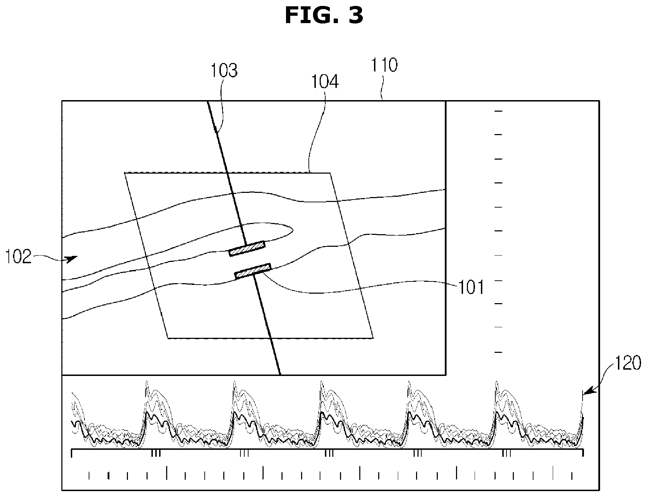

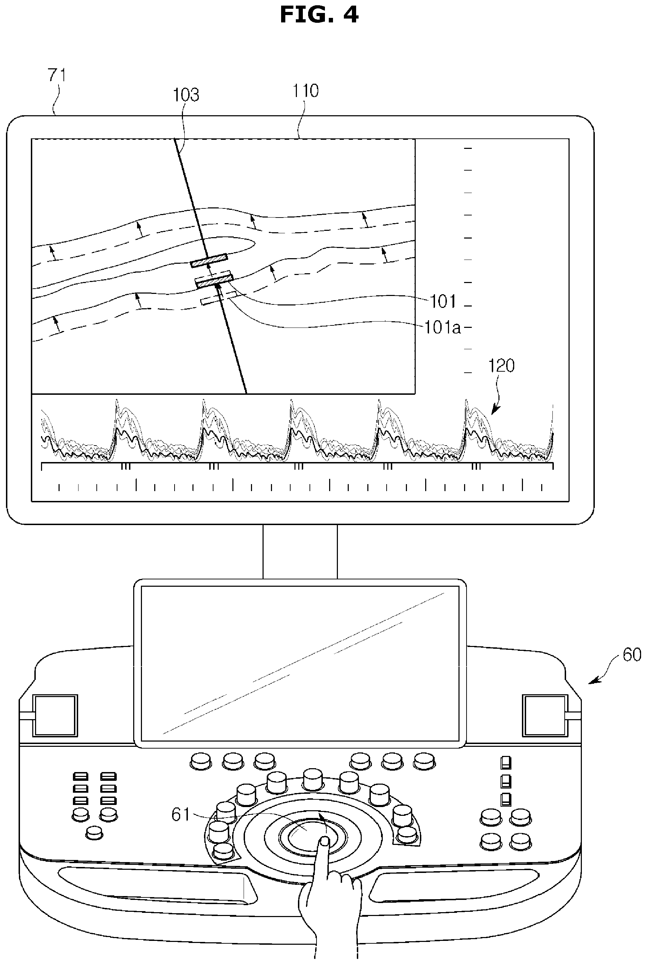

FIGS. 3 and 4 are views illustrating inconvenience of a conventional ultrasonic diagnostic apparatus. To avoid redundant description, description thereof will be described together.

FIG. 3 is a Doppler flow imaging schematically illustrating the blood flow. Particularly, the Doppler flow imaging may be classified into color Doppler determining a color according to a direction of the blood flow and determining a brightness according to a speed of the blood flow, power Doppler more sensitively imaging the blood flow than the color Doppler, wherein a direction of the blood flow is not clear in power Doppler, and spectral Doppler quantifying the blood flow to generate a graph and outputting the graph with the image.

An image of FIG. 3 schematically illustrates the spectral Doppler, and a graph 120 illustrates the speed of the blood flow 102. The speed may be displayed numerically around the graph 120.

An ultrasound imaging 110 of the blood flow 102 shown at the top of the graph 120 includes a Doppler gate 101 corresponding to a region to which Doppler ultrasound is radiated, a guideline 103 including the Doppler gate 101, and a color region of interest (ROI) box 104. The displayed symbol indicated by 101 may also be referred to as a representation of a Doppler gate.

Generally, the ultrasonic diagnostic apparatus searches for the blood flow in a color mode image, in which a color is applied to a brightness mode image, and selects a certain region for determining a speed and a direction of the blood flow so as to generate a Doppler flow imaging. The certain region corresponds to a region of interest (ROI) and the color ROI box 104 is displayed with the Doppler flow imaging, on the ultrasound imaging 110.

Referring to FIG. 4, according to the conventional manner, an ultrasonic diagnostic apparatus recreates the ultrasound imaging 110 due to the movement of the patient during the diagnosis process. In this case, the user has moved the Doppler gate 101 by using the input 60.

Particularly, FIG. 4 illustrates that a user moves the Doppler gate 101 to a position of 101a by using the track ball 61 among components of the input 60. It leads to the inconvenience that moving the position of the Doppler gate 101 manually by using the track ball 61 requires a fine control.

Particularly, as for a portable ultrasonic diagnostic apparatus, a user should touch a display. When the portable ultrasonic diagnostic apparatus is provided with a small size touch screen, user fine control may be interrupted by the small size touch screen. In addition, when the screen is changed as shown in FIG. 4, the user has to convert the image into the brightness mode again, and the Doppler gate must be moved in the Doppler flow imaging in order to find the position of the blood flow, and it causes inconveniences.

In order to solve this inconvenience, the disclosed ultrasonic diagnostic apparatus 100 radiates multi-beams and accumulates data within a range that can be changed. Accordingly, when it is needed to move the Doppler gate 101, the ultrasonic diagnostic apparatus 100 moves the Doppler gate 101 based on the accumulated data. This motion of the Doppler gate may also be referred to as finding a second position of a second Doppler gate.

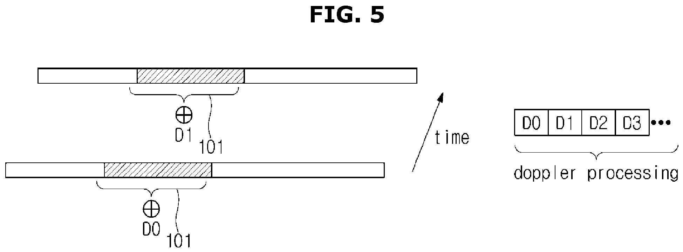

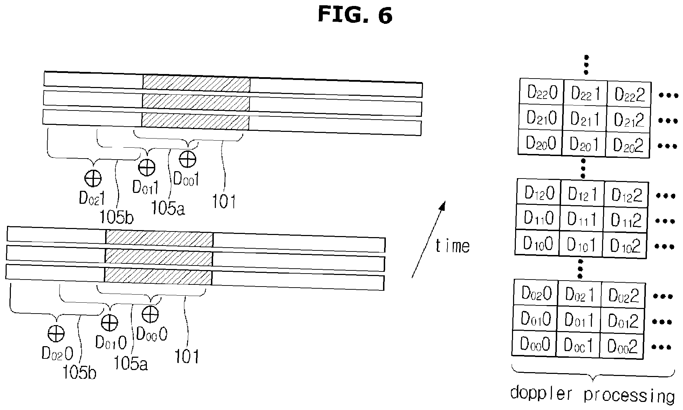

FIGS. 5 and 6 are views illustrating a method of moving a Doppler gate by the ultrasonic diagnostic apparatus. To avoid redundant description, description thereof will be described together.

Referring to FIG. 5, a general Doppler flow imaging periodically radiates an ultrasonic signal to a position corresponding to the Doppler gate 101. The ultrasonic diagnostic apparatus 100 accumulates data D0 and D1 of the Doppler gate 101 region by using a reflected ultrasonic echo signal. That is, the data D0 and D1 generated by the periodically radiated ultrasonic signals are accumulated as time passes, and a Doppler processing is performed based on the accumulated data DO, D1, D2 and D3. Accordingly, the general ultrasonic diagnostic apparatus may display the speed and direction of the blood flow of the object.

According to an embodiment, the ultrasonic diagnostic apparatus 100 simultaneously radiates a plurality of ultrasound signals to a predetermined range around the Doppler gate 101. The ultrasonic diagnostic apparatus 100 receives reflected signals and accumulates a data set within a certain range.

Referring to FIG. 6, the disclosed ultrasonic diagnostic apparatus 100 radiates multi-beams to a certain area other than the Doppler gate 101, that is, data areas 105a and 105b. For example, data measured by a first ultrasonic signal radiated to the area of the Doppler gate 101 is D000. Data measured by second and third ultrasonic signals radiated to the data area 105, which is set other than the Doppler gate 101, are D010 and D020.

As illustrated in FIG. 5, the Doppler flow imaging periodically radiates an ultrasonic signal to detect a change in speed. The ultrasonic diagnostic apparatus 100 also radiates first multi-beams, and then radiates multi-beams again after a predetermined time. The data accumulated by second multi-beams are D001 D011 and D021. As time passes, the range set in the Doppler gate 101 and the range set in the vicinity of the Doppler gate 101 may be accumulated as illustrated in FIG. 6.

The ultrasonic diagnostic apparatus 100 may proceed with the Doppler processing based on the accumulated data, and although the object is moved, the ultrasonic diagnostic apparatus 100 may move the Doppler gate 101 based on the accumulated data.

Accordingly, the ultrasonic diagnostic apparatus 100 may automatically move the Doppler gate 101 without returning to the brightness mode or without the user input.

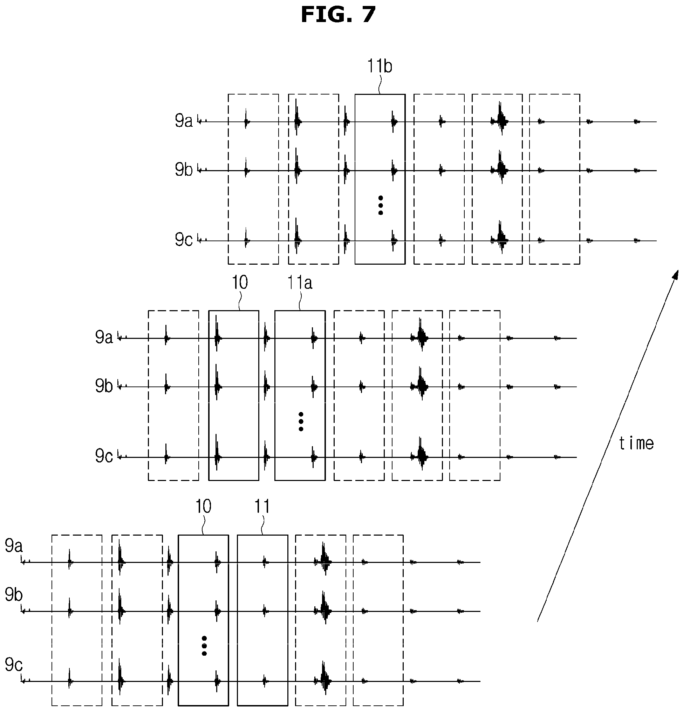

FIG. 7 is a view supplementing the description of FIG. 6.

The conventional ultrasonic diagnostic apparatus radiates a single ultrasonic signal and repeatedly transmits and receives the signals to generate a Doppler flow imaging, as illustrated in FIG. 5. As time passes, the conventional ultrasonic diagnostic apparatus generates the Doppler flow imaging based on data that is accumulated in the first data area 10 corresponding to the Doppler gate 101.

In comparison with the conventional manner, the disclosed ultrasonic diagnostic apparatus 100 radiates three ultrasonic signals 9a, 9b and 9c. In addition, the ultrasonic diagnostic apparatus 100 periodically and repeatedly radiates three ultrasonic signals according to three periods.

As illustrated in FIG. 7, the ultrasonic diagnostic apparatus 100 may accumulate data of the second data area 11, 11a, and 11b as well as the first data area 10 based on the Doppler gate 101.

Meanwhile, when the data stored in the first data area 10 is changed by an event such as the movement of the object, the ultrasonic diagnostic apparatus 100 compares the first data area 10 with the second data area 11, 11a, and 11b. Accordingly, the ultrasound diagnostic apparatus 100 may automatically correct the Doppler gate 101 thereby reducing user inconvenience.

In addition, the size and number of the second data area 11, 11a, and 11b may vary according to the user input or may be set in advance.

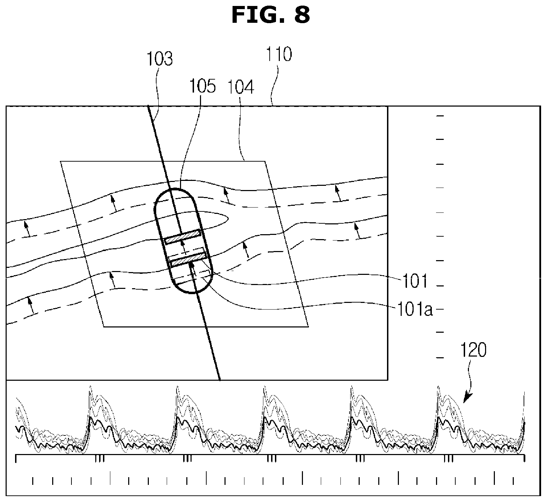

FIG. 8 is a view illustrating an operation of a user interface in accordance with an embodiment. A description of the same parts as those shown FIG. 3 will be omitted.

As mentioned above, the ultrasonic diagnostic apparatus 100 radiates an ultrasonic signal to a predetermined range around the Doppler gate 101 corresponding to the data area 105. The ultrasonic diagnostic apparatus outputs a certain range 105 to allow a user to recognize the data area 105 on the ultrasound imaging 110 as illustrated in FIG. 8.

When the position of the blood flow is changed by the movement of the object, the ultrasonic diagnostic apparatus 100 moves the Doppler gate 101a to a position 101 in the set range 105, along the guide line 103. That is, according to an embodiment, the ultrasonic diagnostic apparatus 100 may vertically move the Doppler gate 101 along the guide line 103.

The certain range 105 may be stored in advance, and may be reset by the user.

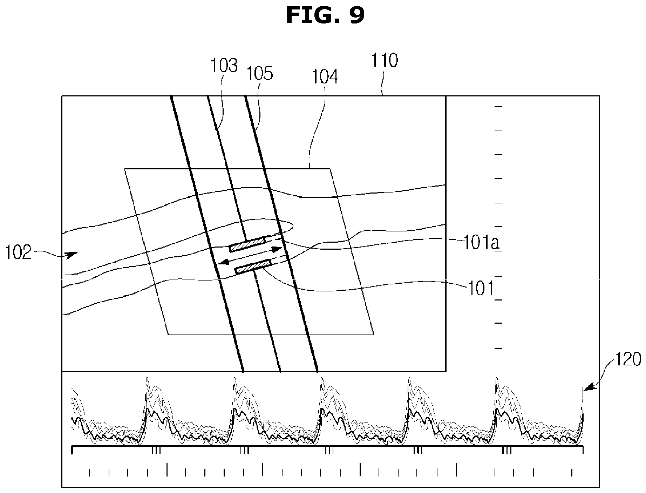

FIG. 9 is a view illustrating an operation of a user interface in accordance with another embodiment.

Unlike FIG. 8, an area 105 to which multi-beams are radiated from the ultrasonic diagnostic apparatus 100 may be designated to be parallel with the guide line 103. In this case, the Doppler gate 101 may be extended or shifted to the left or the right side with respect to a previous position as shown in FIG 9.

That is, in FIG. 9, the Doppler gate 101a placed in the previous position may be moved to the left side, corresponding to the position 101, in the data area 105.

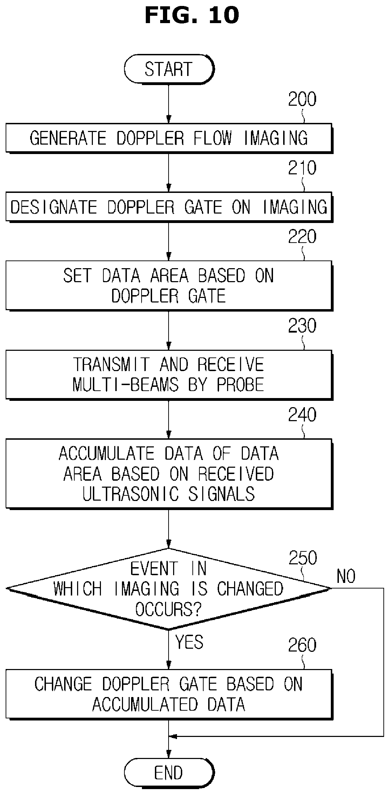

FIG. 10 is a flow chart illustrating an operation in accordance with an embodiment.

Referring to FIG. 10, the ultrasonic diagnostic apparatus 100 generates an image in the Doppler mode, that is, a Doppler flow imaging (200).

As mentioned above, the Doppler flow imaging may be classified into color Doppler determining a color according to a direction of the blood flow and determining a brightness according to a speed of the blood flow, power Doppler more sensitively imaging the blood flow than the color Doppler, wherein a direction of the blood flow is not clear in power Doppler, and spectral Doppler quantifying the blood flow to generate a graph and outputting the graph with the image.

In other words, it is sufficient that the Doppler flow imaging includes the Doppler gate 101 and the Doppler flow imaging corresponds to an image generated by periodically radiating Doppler ultrasound.

A user designates the Doppler gate 101 using the input 60 (210). The Doppler gate 101 may have a different size and position in accordance with input of the user, and the Doppler gate 101 may be pre- set and generated in a portion of the object in motion.

The ultrasonic diagnostic apparatus 100 sets the data area 105 based on the position of the Doppler gate 101, according to the user input (220).

The data area 105 represents a range in which multi ultrasonic signals are radiated to acquire data by having the Doppler gate 101.

The data area may be set by a user, or may include a predetermined certain range. The user may change the size of the data area 105 through the input 60.

The ultrasonic diagnostic apparatus 100 may inform the user of a section in which the data area 105 is set, through the output portion 70.

When the data area 105 is set, the ultrasonic diagnostic apparatus 100 radiates a plurality of Doppler ultrasound signals to the data area 105 and receives the reflected signals (230).

The plurality of Doppler ultrasound signals is periodically radiated and the ultrasonic diagnostic apparatus 100 may determine the speed and direction of the movement of the object based on echo signals which are repeatedly received.

In other words, the ultrasonic diagnostic apparatus 100 accumulates data of the data area 105 other than the Doppler gate 101 based on the received ultrasonic signals (240).

The ultrasonic diagnostic apparatus 100 determines whether an event, in which an image is changed by the movement of the object, occurs (250).

The event may vary. For example, in a state in which the ultrasonic diagnostic apparatus 100 fixes the position of the Doppler gate 101, when a user moves the probe P, the ultrasonic diagnostic apparatus 100 may automatically correct the position of the Doppler gate 101 according to the movement of the probe P. The correction of the position of the Doppler gate may also be described as follows. The probe radiates and echoes are captured and processed with respect to a first depth or first Doppler gate providing information about, for example, blood flow in a blood vessel. The apparatus 100 then automatically, because of for example, motion of the probe or motion of a patient, automatically radiates and captures associated with a second depth (second Doppler gate) providing information again about blood flow in the blood vessel.

Based on the accumulated data, the ultrasonic diagnostic apparatus 100 moves the Doppler gate 101 to a position of data of the data area 105 similar with data collected in a position in which the Doppler gate 101 previously is displayed (260). In other words, he apparatus 100 automatically, because of for example, motion of the probe or motion of a patient, automatically radiates and captures associated with a second depth (second Doppler gate) providing information again about blood flow in the blood vessel.

A range in which the Doppler gate 101 is moved is based on the data area 105. The data area 105 may vary according to the user input, and the ultrasonic diagnostic apparatus 100 may output the size of the data area 105 and a result of movement of the Doppler gate 101 through the user interface.

As for an example of the user interface, the ultrasonic diagnostic apparatus 100 may be ready for an operation start command. That is, when a user inputs a command for moving the Doppler gate 101 and inputs the operation start command, the ultrasonic diagnostic apparatus 100 may move the Doppler gate 101 from a position that is designated by the user, again.

In addition, the ultrasonic diagnostic apparatus 100 may help the user to easily select a Doppler gate by displaying the data area 105 in a variety of color or in a variety of intensity.

A detail example related to the user interface will be described below with reference to the drawings.

In summary, the ultrasonic diagnostic apparatus 100 may radiate a plurality of ultrasonic signals to the data area 105 other than the Doppler gate 101, and accumulate data for correcting the Doppler gate 101 in preparation for a case in which an event occurs. Accordingly, it may be possible to reduce the user inconvenience such as a case in which the user manually moves the Doppler gate, again or a case in which the user searches for the position of the blood flow by changing the mode to the brightness mode.

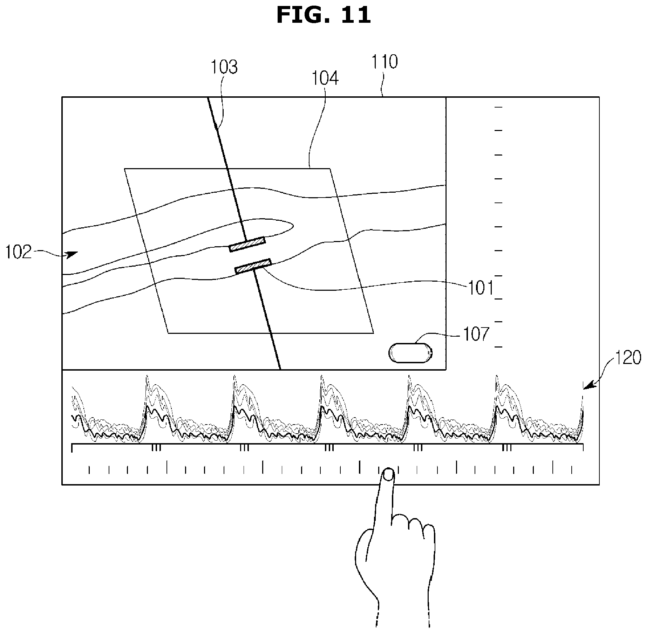

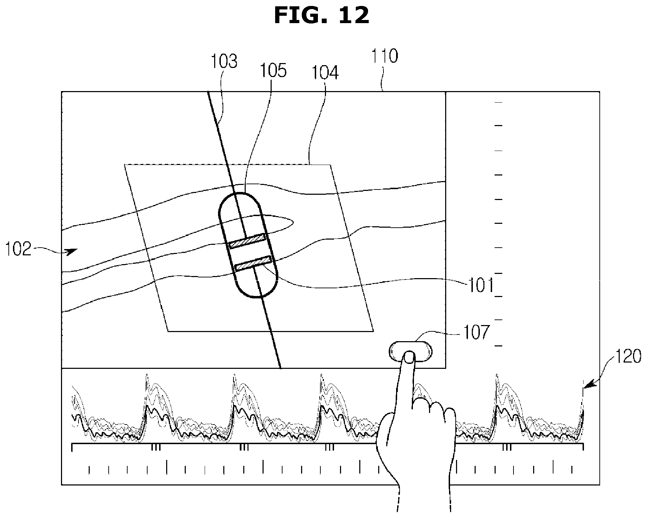

FIGS. 11 and 12 are views illustrating an example related to the user interface. To avoid redundant description, description thereof will be described together.

Referring to FIG. 11, the ultrasonic diagnostic apparatus 100 may output a Doppler flow imaging in the spectral Doppler type. The spectral Doppler imaging may include the ultrasound imaging 110 and the graph 120 indicating the speed of the object by quantifying the speed of the object.

The ultrasound imaging 110 may include a Doppler gate 101 corresponding to a region to which Doppler ultrasound is radiated, a guideline 103 including the Doppler gate 101, a color region of interest (ROI) box 104.

As illustrated in FIG. 11, the ultrasonic diagnostic apparatus 100 may display that the Doppler gate 101 is placed in a portion of the object such as the blood flow.

In addition, as illustrated in FIG. 11, the ultrasonic diagnostic apparatus 100 may display a start button 107, that is, a user interface, configured to execute the above mentioned operation.

According to an embodiment, since the ultrasonic diagnostic apparatus 100 is provided with a touch screen provided in the display, the ultrasonic diagnostic apparatus 100 may receive a touch input of the user.

As illustrated in FIG. 12, the ultrasonic diagnostic apparatus 100 may receive a touch input of the user. That is, when the user touches the start button 107, the ultrasonic diagnostic apparatus 100 may output the data area 105 in the vicinity of the Doppler gate 101 by a predetermined range.

The user may determine that, based on displayed data area 105, the ultrasonic diagnostic apparatus 100 radiates multi ultrasonic signals and accumulates data to correct the Doppler gate 101 according to the occurrence of the event.

Meanwhile, the user may change the data area 105 via the input 60. In addition, the ultrasonic diagnostic apparatus 100 may output the data area 105 in a variety of shapes and positions that is different from FIG. 12. Therefore, there is no limitation in the shape and position of the data area 105.

As is apparent from the above description, according to the proposed ultrasonic diagnostic apparatus and control method thereof, it may be possible automatically correct and move a position of a Doppler gate in a Doppler mode so that a user does not need to search for a position of a target by converting the Doppler mode to another mode, and so that a difficulty in correcting the position of the Doppler gate caused by a manual operation is resolved, and a control method thereof.

Although a few embodiments of the disclosure have been shown and described, it would be appreciated by those skilled in the art that changes may be made in these embodiments without departing from the principles and spirit of the disclosure, the scope of which is defined in the claims and their equivalents.

## Claims

1. An ultrasonic diagnostic apparatus comprising:
a probe configured to radiate ultrasonic signals to an object and configured to receive the reflected ultrasonic signals;
an image processor configured to generate a Doppler flow imaging based on the ultrasonic signals received by the probe;
an output portion configured to display the Doppler flow imaging generated by the image processor and a Doppler gate; and
a controller configured to control the probe so that the probe radiates a first ultrasonic signal based on a position of the Doppler gate, and the probe radiates a second ultrasonic signal to a predetermined range based on the position of the Doppler gate, and configured to move the position of the Doppler gate on the Doppler flow imaging based on the received first ultrasonic signal and the received second ultrasonic signal.

2. The ultrasonic diagnostic apparatus of claim 1, wherein
the controller accumulates data of the first received ultrasonic signal and the second received ultrasonic signal received based on the position and a size of the Doppler gate.

3. The ultrasonic diagnostic apparatus of claim 1, wherein
the controller compares the received first ultrasonic signal with the received second ultrasonic signal.

4. The ultrasonic diagnostic apparatus of claim 2, wherein
the controller proceeds with a Doppler processing based on the accumulated data and generates the Doppler flow imaging.

5. The ultrasonic diagnostic apparatus of claim 3, wherein
the controller moves the position of the Doppler gate based on a result of the comparison.

6. The ultrasonic diagnostic apparatus of claim 1, wherein
the controller moves the Doppler gate from a position to which the first ultrasonic signal is radiated, to an area to which the second ultrasonic signal is radiated.

7. The ultrasonic diagnostic apparatus of claim 1, wherein
the controller changes the size of the Doppler gate based on an area to which the second ultrasonic signal is received.

8. The ultrasonic diagnostic apparatus of claim 1, wherein
the predetermined range comprises a guide line comprising the Doppler gate.

9. The ultrasonic diagnostic apparatus of claim 1, wherein
the output portion outputs at least one of a mark and a sound indicating that the Doppler gate is moved.

10. The ultrasonic diagnostic apparatus of claim 1, wherein
the output portion outputs the Doppler flow imaging, and an interface configured to receive an instruction from an operation of the controller.

11. A control method of an ultrasonic diagnostic apparatus comprising:
generating a Doppler flow imaging based on an ultrasonic signal that is radiated to an object and then reflected from the object;
displaying the generated Doppler flow imaging and a Doppler gate; and
moving a position of the Doppler gate on the Doppler flow imaging based on a first ultrasonic signal placed in the Doppler gate and a second ultrasonic signal radiated to a predetermined range with respect to the position of the Doppler gate.

12. The control method of claim 11, wherein
the moving comprises accumulating data of the first received ultrasonic signal and the second received ultrasonic signal based on the position and a size of the Doppler gate.

13. The control method of claim 11, wherein
the moving comprises moving the position of the Doppler gate by comparing the received first ultrasonic signal with the received second ultrasonic signal.

14. The control method of claim 12, wherein
the moving further comprises proceeding with a Doppler processing based on the accumulated data, generating the Doppler flow imaging and moving the Doppler gate.

15. The control method of claim 11, wherein
the moving comprises moving the Doppler gate from a position to which the first ultrasonic signal is radiated, to an area to which the second ultrasonic signal is radiated.
